# EUROPEAN PATENT SPECIFICATION

(11) **EP 4 368 233 B1**
(45) Date of publication and mention of the grant of the patent: **16.09.2026**
(21) Application number: 23764217.8
(22) Date of filing: 01.06.2023
(51) Int. Cl.: A61M 37/00, A24F 40/42, A24B 15/167, A24B 15/24, A61K 31/465

(54) **ELECTRONIC DEVICE FOR RELEASING EMULSION**
ELEKTRONISCHE VORRICHTUNG ZUR FREISETZUNG EINER EMULSION
DISPOSITIF ÉLECTRONIQUE POUR LIBÉRER UNE ÉMULSION

(30) Priority: 20.09.2022 KR 20220118761
(43) Date of publication of application: 15.05.2024
(73) Proprietor: KT&G Corporation, Daedeok-gu Daejeon 34337 (KR)
(72) Inventor: KIM, Jae Hyun, Daejeon 34128 (KR); KANG, Won Hyuk, Daejeon 34128 (KR); JEONG, Minseok, Daejeon 34128 (KR); CHUNG, Tae Young, Daejeon 34128 (KR)
(74) Representative: Ter Meer Steinmeister & Partner
(86) International application number: PCT/KR2023/007546
(87) International publication number: WO 2024/063246

(56) References cited:
- EP-B1- 2 227 286
- JP-A- 2007 529 502
- JP-A- 2014 213 425
- KR-A- 20060 063 122
- KR-A- 900 005 911
- US-A1- 2011 264 048
- US-A1- 2011 295 230
- US-A1- 2022 096 806
- US-A1- 2022 193 385

## Description

### Technical Field

The following embodiments relate to technology for releasing emulsion through an electronic device, and more particularly, to an electronic device attached to a skin of a user.

### Background Art

These days, there is a gradual rise in the demand for electronic cigarettes. The rising demand for electronic cigarettes has accelerated the continued development of electronic cigarette-related functions. The electronic cigarette-related functions may include, in particular, functions according to types and characteristics of electronic cigarettes. Such functions include releasing substances to the skin of a user. US 2011/295230 A1 and US 2011/264048 A1 both disclose micro-needle devices wherein a substance heated presses a membrane against a puncturing member, releasing an active substance through a micro-needle.

### Disclosure of the Invention

### Technical Solutions

According to an embodiment, an electronic device for releasing emulsion includes a cartridge accommodation portion configured to accommodate a cartridge containing emulsion therein, a crushing circuit including a crushing element configured to crush at least a portion of the cartridge disposed in the cartridge accommodation portion, a processor configured to generate a crushing signal, and a transmission circuit configured to transmit the crushing signal to the crushing circuit, wherein emulsion is be released to an outside of the electronic device by a microneedle, wherein the emulsion is exposed as at least the portion of the cartridge is crushed.

The crushing element is formed in the substrate of the crushing circuit to penetrate the substrate, and the crushing element forms a bump on a second surface of the substrate while forming a closed circuit with a first transmission element and a second transmission element of the transmission circuit on a first surface of the substrate.

A material of the crushing element may include a material that generates heat by the crushing signal, and at least the portion of the cartridge may be crushed by heat generated by the crushing element.

The material of the crushing element may be a metal having a specific resistance.

At least the portion of the cartridge crushed by the heat may be a thin-film cell.

The crushing circuit may further include an elastic element disposed between one side of a substrate of the crushing circuit and the cartridge.

The crushing circuit may further include an emulsion release element attached to the elastic element, and the elastic element and the emulsion release element may be configured to move the emulsion in the direction of the microneedle when at least the portion of the cartridge is crushed by the crushing element.

The electronic device may further include an indicator indicating an amount of emulsion to be released.

The electronic device may further include a button configured to receive, from a user, an amount of emulsion to be released.

The processor may be configured to, when the cartridge includes a plurality of blocks, each containing emulsion, generate the crushing signal to crush one or more blocks of the plurality of blocks. The crushing circuit may include a plurality of crushing elements, and the plurality of crushing elements may respectively correspond to the plurality of blocks.

According to an example not being part of the invention, a method of releasing emulsion, performed by an electronic device, includes determining a first block among a plurality of blocks of a cartridge included in the electronic device, generating a first crushing signal for crushing at least a portion of the first block, and applying the first crushing signal to a first crushing element of a crushing circuit corresponding to the first block through a transmission circuit, wherein as at least a portion of the first block is crushed by heat generated by the first crushing element, the emulsion in the first block is released.

### Brief Description of Drawings

FIG. 1 illustrates a schematic diagram of an emulsion release system according to an example.
FIG. 2 illustrates a block diagram of an electronic device according to an embodiment.
FIG. 3A illustrates a connection relationship between a transmission circuit and a crushing circuit according to an embodiment.
FIG. 3B illustrates a connection relationship among a crushing circuit, a cartridge accommodation portion, and a microneedle portion according to an example.
FIG. 4 illustrates a connection relationship between a crushing circuit including an elastic element and a cartridge in a cartridge accommodation portion according to an example.
FIG. 5 illustrates a connection relationship between a cartridge accommodation portion and a microneedle portion according to an example.
FIG. 6 illustrates a crushing circuit including an infrared (IR) lamp, according to example.
FIG. 7 illustrates a cross-sectional view of an electronic device capable of transmitting external IR light in the direction of a skin of a user according to an example.
FIG. 8 illustrates a crushing circuit including an ultrasonic vibrator, according to an example.
FIG. 9 illustrates a cross-sectional view of an electronic device capable of transmitting external ultrasound in the direction of a skin of a user according to an example.
FIG. 10 is a flowchart of a method (not claimed) of releasing emulsion.
FIG. 11 is a flowchart of a method (not claimed) of generating a crushing signal based on a user input, according to an example.
FIG. 12 is a flowchart of a method (not claimed) of controlling an IR lamp, according to an example
FIG. 13 is a flowchart of a method (not claimed) of controlling an ultrasonic vibrator, according to an example.

### Best Mode for Carrying Out the Invention

Although terms, such as first, second, and the like are used to describe various components, the components are not limited to the terms. These terms should be used only to distinguish one component from another component. For example, a first component may be referred to as a second component, and similarly the second component may also be referred to as the first component.

It should be noted that, if one component is described as being "connected," "coupled," or "joined" to another component, a third component may be "connected," "coupled," and "joined" between the first and second components, although the first component may be directly connected, coupled, or joined to the second component.

The singular forms "a," "an," and "the" are intended to include the plural forms as well, unless the context clearly indicates otherwise. It will be further understood that the terms "comprises/comprising" and/or "includes/including," when used herein, specify the presence of stated features, integers, steps, operations, elements, components, or combinations thereof, but do not preclude the presence or addition of one or more other features, integers, steps, operations, elements, components and/or combinations thereof.

Unless otherwise defined, all terms, including technical and scientific terms, used herein have the same meaning as commonly understood by one of ordinary skill in the art to which the present disclosure pertains. Terms, such as those defined in commonly used dictionaries, are to be interpreted as having a meaning that is consistent with their meaning in the context of the relevant art, and are not to be interpreted in an idealized or overly formal sense unless expressly so defined herein.

Hereinafter, embodiments are described in detail with reference to the accompanying drawings. When describing the embodiments with reference to the accompanying drawings, like reference numerals refer to like elements and a repeated description related thereto will be omitted.

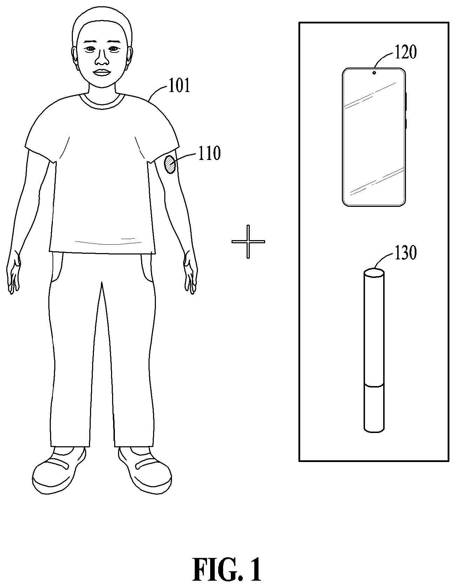

FIG. 1 illustrates a schematic diagram of an emulsion release system according to an example.

Referring to FIG. 1, the emulsion release system may include an electronic device 110 and an external device. For example, the external device may include a user terminal 120 (e.g., a smartphone) or a smokeless inhalation device 130 (e.g., an electronic cigarette or an inhaler).

According to an embodiment, the electronic device 110 may be attached to a part of the body of a user 101 and release emulsion (e.g., a liquid composition) onto or within the skin of the user 101. The emulsion released to the skin of the user 101 may be absorbed into the body of the user 101 through the skin. For example, the emulsion may include a medicinal component required for the user to treat a disease that the user 101 has. For example, the emulsion may include various vitamin components and mineral components. For example, the emulsion may include a component (e.g., nicotine) for the preferences of the user 101. For example, the acidity of the emulsion may be a value (e.g., between pH 7.0 and pH 8.0) that minimizes user irritation. When the pH of the emulsion is high, the emulsion may cause skin irritation due to its alkaline nature. When the pH of the emulsion is low, the emulsion may cause skin irritation due to its acidic nature. For example, the emulsion may be a liquid material containing water-soluble and lipid-soluble materials mixed with a surfactant in a predetermined ratio. The materials contained in the emulsion may be mixed to be evenly dispersed in the form of nano-sized droplets. In the present disclosure, the acidity of the emulsion is exemplified to be between pH 7.0 and pH 8.0. However, the acidity of the emulsion is not limited to the disclosed embodiment and may be adjusted to higher or lower values, considering skin absorption rate.

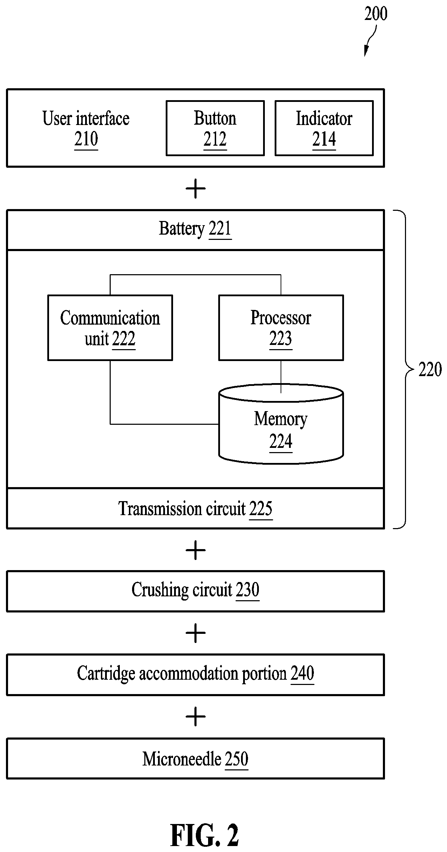

The electronic device 110 is described in detail below with reference to FIGS. 2 to 9.

According to an example, the electronic device 110 may be used independently as a standalone device. For example, the user 101 may directly input a control signal to the electronic device 110, and the electronic device 110 may control an operation of the electronic device 110 based on the control signal of the user 101.

According to an example, the electronic device 110 may establish a wireless communication connection with the user terminal 120 and receive the control signal from the user 101 through the user terminal 120.

According to an example, the electronic device 110 may establish a wireless communication connection with the smokeless inhalation device 130 and receive the control signal from the smokeless inhalation device 130. For example, when the user 101 uses the smokeless inhalation device 130, the smokeless inhalation device 130 may transmit a control signal corresponding to motion of the user to the electronic device 110. Upon receiving the control signal corresponding to the motion of the user, the electronic device 110 may release the emulsion to the user 101 based on the control signal.

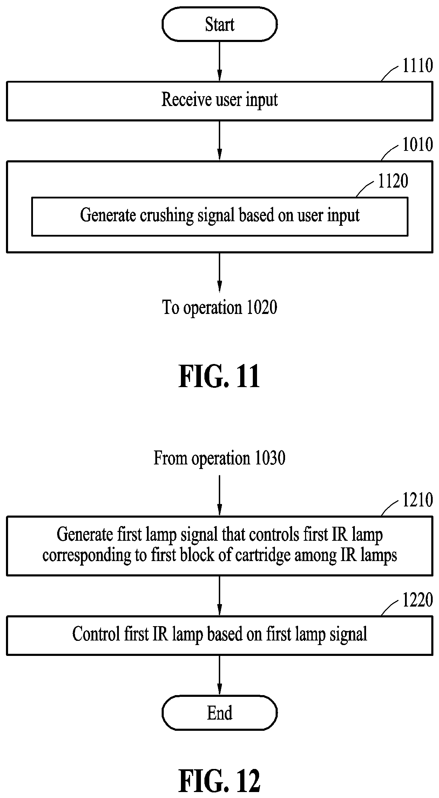

Various embodiments of the electronic device 110 receiving a user input are described in detail below with reference to FIG. 11.

FIG. 2 illustrates a block diagram of an electronic device according to an embodiment.

According to an embodiment, an electronic device 200 (e.g., the electronic device 110 of FIG. 1) may include a user interface 210, a controller 220, a crushing circuit 230, a cartridge accommodation portion 240, and a microneedle portion 250. The electronic device 200 may further include a fixing portion (e.g., a band, sticker, or layer) that provides a fixing force, allowing the electronic device 200 to be attached to a part of the body of a user. The fixing portion may be of a flexible type to enhance skin adherence. The material of the fixing portion may be a material (e.g., a porous polymer material) that does not cause skin irritation.

According to an embodiment, the user interface 210 may include a button 212 for physically receiving a user input and an indicator 214 (e.g., a light-emitting diode (LED)) for visually indicating a state of the electronic device 200. For example, the button 212 may receive, from the user, the amount of emulsion to be released. For example, the indicator 214 may indicate the amount of emulsion to be released. The user interface 210 may be disposed outside a housing of the electronic device 200.

According to an embodiment, the controller 220 may include a battery 221 that may supply power to the electronic device 200, a communication unit 222, a processor 223, a memory 224, and a transmission circuit 225. For example, the controller 220 may include a printed circuit board (PCB) that includes at least some of the battery 221, the communication unit 222, the processor 223, the memory 224, and the transmission circuit 225.

The communication unit 222 may be connected to the processor 223 and the memory 224 and transmit and receive data to and from the processor 223 and the memory 224. The communication unit 222 may transmit and receive data to and from other external devices (e.g., the user terminal 120 or the smokeless inhalation device 130 of FIG. 1) through short-range wireless communication. Hereinafter, transmitting and receiving "A" may refer to transmitting and receiving "information or data indicating A".

The communication unit 222 may be implemented as circuitry in the controller 220. For example, the communication unit 222 may include an internal bus and an external bus. In another example, the communication unit 222 may be an element that connects the controller 220 (or the electronic device 200) to an external device. The communication unit 222 may be an interface. The communication unit 222 may receive data from the external device and transmit the data to the processor 223 and the memory 224.

The processor 223 may process the data received by the communication unit 222 and data stored in the memory 224. A "processor" may be a hardware-implemented data processing device having a physically structured circuit to execute desired operations. The desired operations may include, for example, code or instructions included in a program. The hardware-implemented data processing device may include, for example, a microprocessor, a central processing unit (CPU), a processor core, a multi-core processor, a multiprocessor, an application-specific integrated circuit (ASIC), and a field-programmable gate array (FPGA).

The processor 223 may execute computer-readable code (e.g., software) stored in a memory (e.g., the memory 224) and instructions triggered by the processor 223.

The memory 224 may store data received by the communication unit 222 and data processed by the processor 223. For example, the memory 224 may store a program (or an application or software). The program to be stored may be a set of syntax that is coded and executable by the processor 223 to control the electronic device 200.

According to an aspect, the memory 224 may include at least one volatile memory, non-volatile memory, random-access memory (RAM), flash memory, a hard disk drive, and an optical disc drive.

The memory 224 may store an instruction set (e.g., software) for operating the controller 220. The instruction set for operating the controller 220 may be executed by the processor 223.

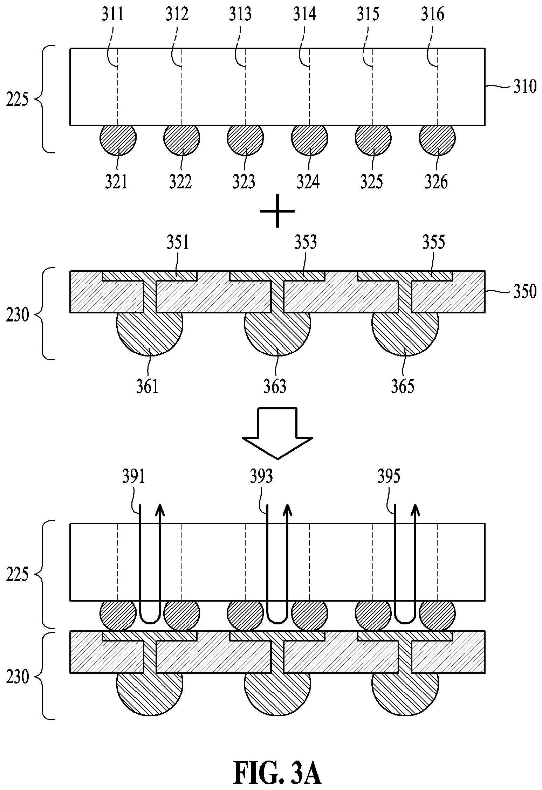

The transmission circuit 225 may transmit a signal (e.g., a crushing signal) generated by the processor 223 to the crushing circuit 230. The transmission circuit 225 is described in detail below with reference to FIG. 3A.

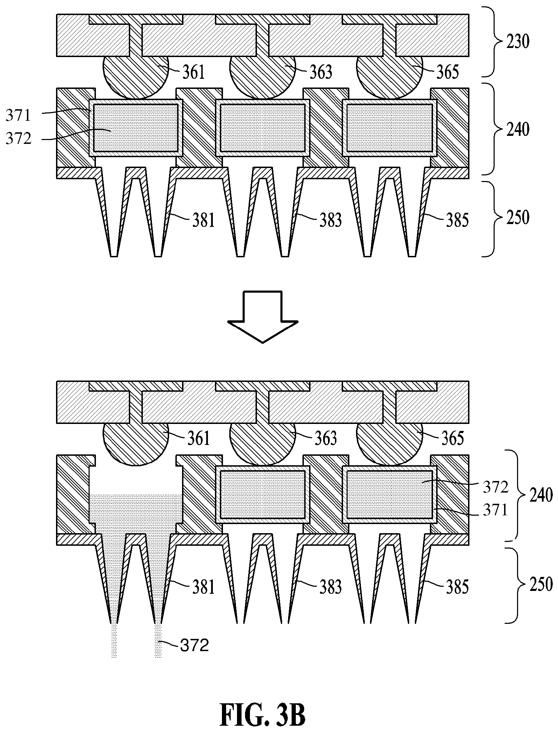

According to an embodiment, the crushing circuit 230 may crush at least a portion of the cartridge in the cartridge accommodation portion 240 based on the crushing signal transmitted through the transmission circuit 255. For example, a crushing element of the crushing circuit 230 may generate heat by the crushing signal, and at least the portion of the cartridge may be crushed by the heat. An embodiment in which at least the portion of the cartridge is crushed by the crushing element is described in detail below with reference to FIG. 3B.

According to an embodiment, the cartridge accommodation portion 240 may be configured to accommodate the cartridge therein. For example, the cartridge accommodation portion 240 may be formed such that one side of the cartridge disposed in the cartridge accommodation portion 240 comes into contact with the crushing element of the crushing circuit 230, while the other side of the cartridge is disposed in the direction of the microneedle portion 250. For example, the cartridge may include one or more blocks, and each of the blocks of the cartridge may be separately inserted into the cartridge accommodation portion 240. The cartridge or a block of the cartridge may be formed based on a thin-film cell that includes emulsion therein. For example, the thin-film cell of the cartridge may be manufactured based on a material (e.g., a polymer composite material or bio-based material) that may be crushed by heat. The emulsion within one block of the cartridge may contain an amount of material equivalent to that of a single typical cigarette. The structure of the cartridge accommodation portion 240 is described in detail below with reference to FIG. 3B.

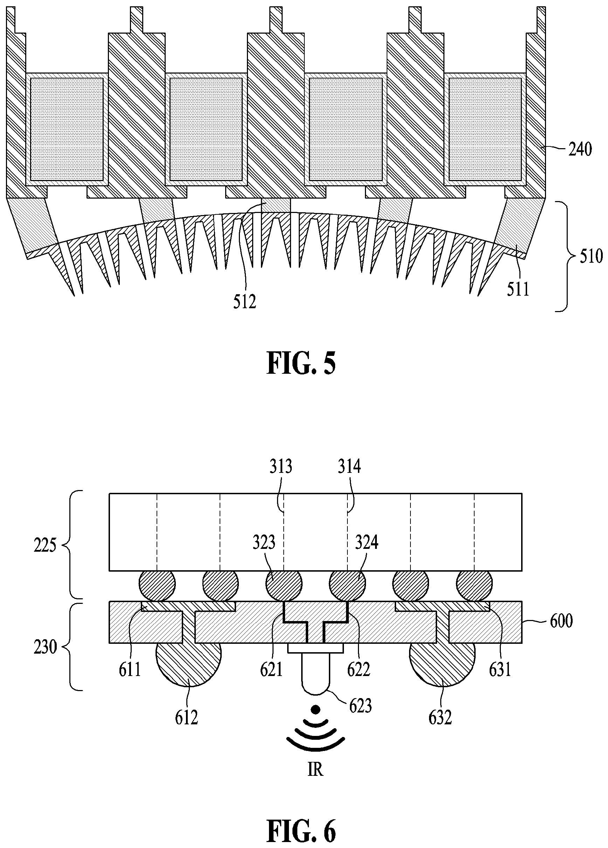

According to an embodiment, the microneedle portion 250 may include a plurality of microneedles. When a microneedle is attached to the skin of a user, the length of the microneedle may allow the microneedle to reach a dermis layer. For example, when the emulsion in the cartridge is exposed to the outside as the cartridge is crushed in the cartridge accommodation portion 240, the emulsion may be released into the dermis layer of the user through the microneedle portion 250. The emulsion released into the dermis layer may be absorbed into capillaries. The microneedle portion 250 may have a structure that may adhere well to the skin of the user. The structure of the microneedle portion 250 is described in detail below with reference to FIG. 5.

FIG. 3A illustrates a connection relationship between a transmission circuit and a crushing circuit according to an embodiment.

According to an embodiment, the transmission circuit 225 of FIG. 2 may include a substrate 310, wires 311, 312, 313, 314, 315, and 316 that pass through the substrate 310, and transmission elements 321, 322, 323, 324, 325, and 326 connected to the wires 311, 312, 313, 314, 315, and 316, respectively. For example, the transmission elements 321, 322, 323, 324, 325, and 326 may be in the form of a ball grid array (BGA). For example, the processor 223 may be positioned on the substrate 310, and pins of the processor 223 may be connected to the wires 311, 312, 313, 314, 315, and 316.

According to an embodiment, the crushing circuit 230 of FIG. 2 may include a substrate 350 and crushing elements formed to pass through the substrate 350. The crushing elements may include a plate and a bump. For example, a first crushing element may include a first plate 351 and a first bump 361, a second crushing element may include a second plate 353 and a second bump 363, and a third crushing element may include a third plate 355 and a third bump 365. A plate may be formed on a first surface of the substrate 350, and a bump electrically connected to the plate may be formed on a second surface of the substrate 350.

According to an embodiment, when the transmission circuit 225 is physically coupled to the crushing circuit 230, the first plate 351 of the first crushing element may be electrically connected to the first transmission element 321 and the second transmission element 322 of the transmission circuit 225. For example, the first plate 351 of the first crushing element may form a first closed circuit 391 with the first transmission element 321 and the second transmission element 322 of the transmission circuit 225 on the first surface of the substrate 350. For example, the second plate 353 of the second crushing element may form a second closed circuit 393 with the third transmission element 323 and the fourth transmission element 324 of the transmission circuit 225 on the first surface of the substrate 350. For example, the third plate 355 of the third crushing element may form a third closed circuit 395 with the fifth transmission element 325 and the sixth transmission element 326 of the transmission circuit 225 on the first surface of the substrate 350.

According to an embodiment, the material of the first crushing element may include a material that generates heat by a signal provided to a closed circuit. When power is supplied through the first closed circuit 391, the first crushing element may act as a resistance, resulting in the generation of heat in the first crushing element. For example, the material of the first crushing element may be a metal (e.g., copper) having a specific resistance.

FIG. 3B illustrates a connection relationship among a crushing circuit, a cartridge accommodation portion, and a microneedle portion according to an example.

According to an embodiment, a cartridge including a plurality of blocks may be inserted into the cartridge accommodation portion 240. For example, a first block 371 of the plurality of blocks may include a thin-film cell including emulsion 372 therein. Components of an emulsion included in each of the plurality of blocks may all be the same, or some components may be different.

According to an embodiment, while the cartridge is inserted into the cartridge accommodation portion 240, the crushing circuit 230 may be disposed on the cartridge accommodation portion 240. For example, the crushing circuit 230 may be coupled to the cartridge accommodation portion 240 such that the bumps 361, 363, and 365 of the crushing circuit 230 and thin-film cells of the cartridge contact each other. A plurality of crushing elements (e.g., the bumps 361, 363, and 365) may respectively correspond to the plurality of blocks of the cartridge in position.

According to an embodiment, the cartridge accommodation portion 240 may be coupled to the microneedle portion 250. For example, the cartridge accommodation portion 240 may be coupled to the microneedle portion 250 such that the emulsion may be released to the outside through a microneedle when the emulsion is exposed in the cartridge accommodation portion 240. For example, the microneedle portion 250 may include a plurality of microneedles 381, 383, and 385.

According to an embodiment, power may be supplied to the first crushing element or the first bump 361 through the first closed circuit 391 described above with reference to FIG. 3A. In this case, the first bump 361 may generate heat, and the generated heat may heat the first block 371 of the cartridge. When the first block 371 is exposed to heat beyond its heat resistance limit, the thin-film cell of the first block 371 may be crushed. When the thin-film cell of the first block 371 is crushed, the emulsion 372 in the thin-film cell may be released to the outside through the first microneedle 381 corresponding to the first block 371.

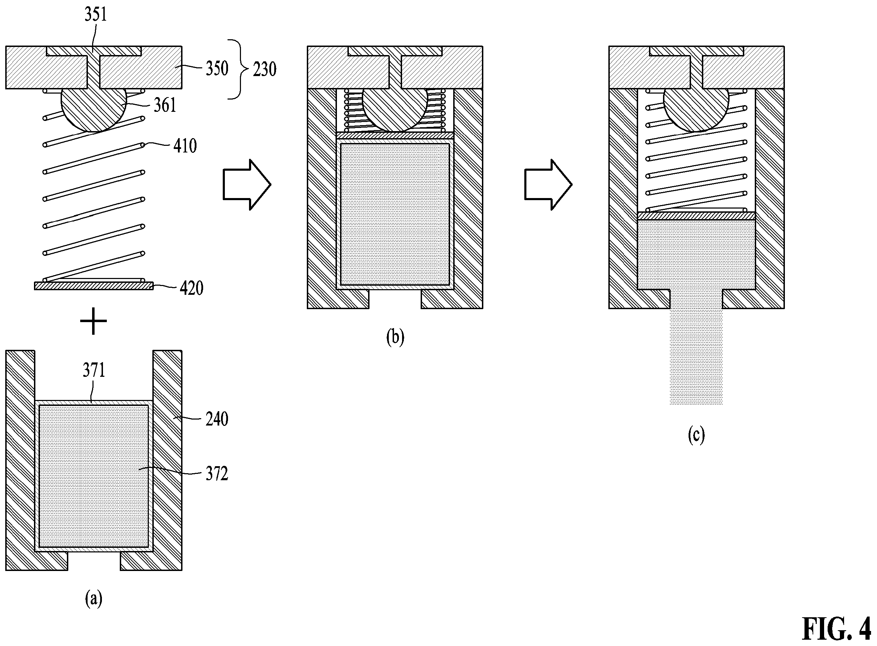

FIG. 4 illustrates a connection relationship between a crushing circuit including an elastic element and a cartridge in a cartridge accommodation portion according to an example.

According to an embodiment, the crushing circuit 230, described above with reference to FIGS. 2 and 3, may further include an elastic element (e.g., a first elastic element 410) and an emulsion release element (e.g., a first emulsion release element 420) in addition to a plate (e.g., the first plate 351) and a bump (e.g., the first bump 361). The elastic element may be disposed between one side (e.g., the bottom surface) of the substrate 350 of the crushing circuit 230 and a cartridge (e.g., the first block 371). For example, the elastic element may be a spring. The emulsion release element may be attached to the elastic element. For example, the material of the emulsion release element may include a material with high thermal conductivity.

According to an embodiment, in the course of coupling the crushing circuit 230 to the cartridge accommodation portion 240, the first elastic element 410 and the first emulsion release element 420 may be compressed by the first block 371 in the cartridge accommodation portion 240 in the direction of the first bump 361. For example, when the crushing circuit 230 is completely coupled to the cartridge accommodation portion 240, the first bump 361 and the first emulsion release element 420 contact each other, and the first emulsion release element 420 and the upper end of the first block 371 may contact each other.

According to an embodiment, when the first bump 361 generates heat, the generated heat may heat the first emulsion release element 420. The first block 371 may be crushed by the first emulsion release element 420 that is heated, and accordingly, the emulsion 372 in the first block 371 may be released to the outside through a microneedle. As the first block 371 is crushed, the first elastic element 410 may push the first emulsion release element 420 outward, and the emulsion 372 in the cartridge accommodation portion 240 may be released to the outside due to the movement of the first emulsion release element 420. In other words, when at least a portion (e.g., the first block 371) of the cartridge is crushed by the first crushing element (e.g., the first bump 361), the first elastic element 410 and the first emulsion release element 420 may move the emulsion 372 in the direction of the microneedle.

According to an embodiment, when the first emulsion release element 420 fills an end surface of the cartridge accommodation portion 240, leakage may be prevented since the emulsion 372 does not flow in the direction of the first bump 361. Furthermore, regardless of the placement or orientation of the electronic device 200, the emulsion 372 may be pushed in the direction of the microneedle.

FIG. 5 illustrates a connection relationship between a cartridge accommodation portion and a microneedle portion according to an example.

According to an embodiment, a microneedle portion 510 (e.g., the microneedle portion 250 of FIG. 2) may include a plurality of connecting units disposed between the cartridge accommodation portion 240 and a plurality of microneedles. For example, the length (or height) of each of the plurality of connecting units may vary depending on its placement location. For example, an outermost connecting unit 511 may be the longest among the rest of the connecting units, and a central connecting unit 512 may be the shortest. Due to the structure described above, the microneedle portion 510 may have an inwardly concave shape. When the microneedle portion 510 has an inwardly concave shape, the adhesion to the skin of a user may be enhanced. For example, the plurality of connecting units and the plurality of microneedles of the microneedle portion 510, which may be made from elastic materials, may adhere to the skin of the user.

FIG. 6 illustrates a crushing circuit including an infrared (IR) lamp, according to example.

According to an embodiment, the crushing circuit 230 described above with reference to FIG. 2 may include a substrate 600 and crushing elements formed to pass through the substrate 600. The crushing elements may include a plate and a bump. For example, a first crushing element may include a first plate 611 and a first bump 612 and a second crushing element may include a second plate 631 and a second bump 632. The plate may be formed on a first surface of the substrate 600, and the bump electrically connected to the plate may be formed on a second surface of the substrate 600.

According to an embodiment, the crushing circuit 230 may further include a first electrode 621, a second electrode 622, and an IR lamp 623 that emits IR light. The IR lamp 623 may be connected to the first electrode 621 and the second electrode 622. For example, the first electrode 621 and the second electrode 622 may be electrically and respectively connected to the third transmission element 323 and the fourth transmission element 324 of the transmission circuit 225 described above with reference to FIG. 3A. For example. the processor 223 may supply power to a closed circuit formed through the wires 313 and 314, the third transmission element 323, the fourth transmission element 324, the first electrode 621, the second electrode 622, and the IR lamp 623. When power is supplied to the closed circuit, the IR lamp 623 may emit IR light. More particularly, the emitted IR light may be near-IR (NIR). When NIR is irradiated to the skin of a user, NIR may reach a dermis layer of the skin.

According to an embodiment, when NIR is irradiated to the skin of the user, the absorption rate or absorption speed of the emulsion released to the skin may increase. For example, NIR may generate heat on the skin, and the generated heat may increase the absorption rate or absorption speed of the emulsion. For example, the heat generated by NIR may destroy nano-capsules in the emulsion in the dermis layer, allowing substances in the nano-capsules to be absorbed into capillaries.

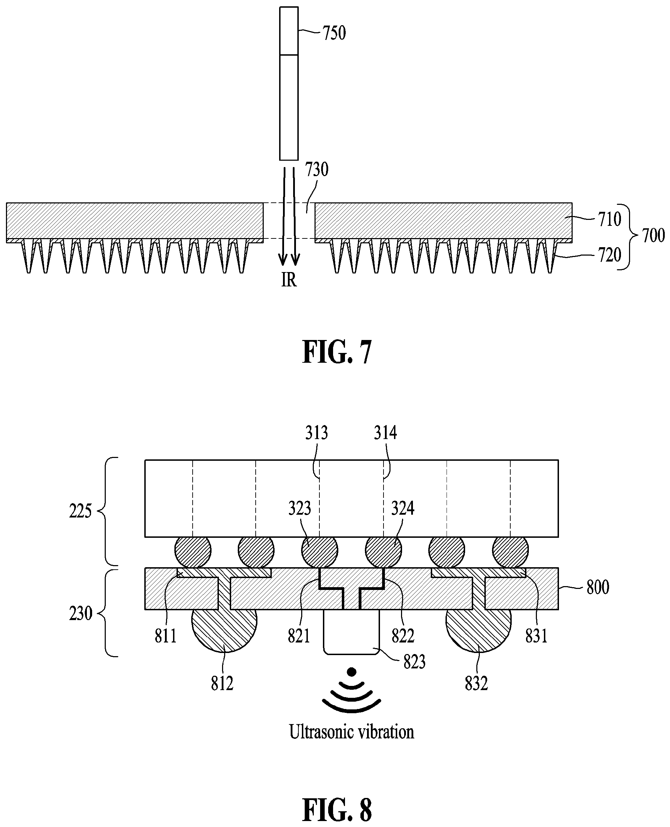

FIG. 7 illustrates a cross-sectional view of an electronic device capable of transmitting external IR light in the direction of the skin of a user, according to an example.

According to an embodiment, an electronic device 700 (e.g., the electronic device 100 of FIG. 1 or the electronic device 200 of FIG. 2) may include a body portion 710 and a microneedle portion 720. For example, the body portion 710 may include the user interface 210, the controller 220, the crushing circuit 230, and the cartridge accommodation portion 240.

According to an embodiment, the electronic device 700 may include a light transmission element 730 through which IR light may pass from one side (e.g., the upper surface) to the other side (e.g., the bottom surface) of the electronic device 700. For example, the shape of the light transmission element 730 may be a hole that penetrates the body portion 710 and the microneedle portion 720. For example, the light transmission element 730 may be an element made of a transparent material formed in the body portion 710 and the microneedle portion 720.

According to an embodiment, a user may irradiate IR light emitted by an IR emission device 750 onto one side of the electronic device 700. For example, the IR emission device 750 may be an electronic cigarette or an inhaler. IR light emitted by the IR emission device 750 may reach the other side of the electronic device 700 through the light transmission element 730 of the electronic device 700. In a state in which the electronic device 700 is attached to the skin of a user, when the user irradiates IR light onto the upper surface of the electronic device 700 using the IR emission device 750, the emitted IR light may reach the bottom surface of the electronic device 700 and thus reach the skin of the user.

FIG. 8 illustrates a crushing circuit including an ultrasonic vibrator, according to an example.

According to an embodiment, the crushing circuit 230 described above with reference to FIG. 2 may include a substrate 800 and crushing elements formed to pass through the substrate 800. The crushing elements may include a plate and a bump. For example, a first crushing element may include a first plate 811 and a first bump 812 and a second crushing element may include a second plate 831 and a second bump 832. The plate may be formed on a first surface of the substrate 800, and the bump electrically connected to the plate may be formed on a second surface of the substrate 800.

According to an embodiment, the crushing circuit 230 may further include a first electrode 821, a second electrode 822, and an ultrasonic vibrator 823 emitting ultrasound or ultrasonic vibrations. The ultrasonic vibrator 823 may be connected to the first electrode 821 and the second electrode 822. For example, the first electrode 821 and the second electrode 822 may be electrically and respectively connected to the third transmission element 323 and the fourth transmission element 324 of the transmission circuit 225 described above with reference to FIG. 3A. For example, the processor 223 may supply power to a closed circuit formed through the wires 313 and 314, the third transmission element 323, the fourth transmission element 324, the first electrode 821, the second electrode 822, and the ultrasonic vibrator 823. When power is supplied to the closed circuit, the ultrasonic vibrator 823 may emit ultrasound or ultrasonic vibrations.

According to an embodiment, the sonic energy of ultrasound or ultrasonic vibrations emitted onto the skin of a user may be converted into heat, and the heat may vaporize moisture within cells. The vaporized moisture within the cells may exert pressure on cellular tissue, causing spaces between the cells to expand. The expanded spaces between the cells may facilitate the absorption of emulsion.

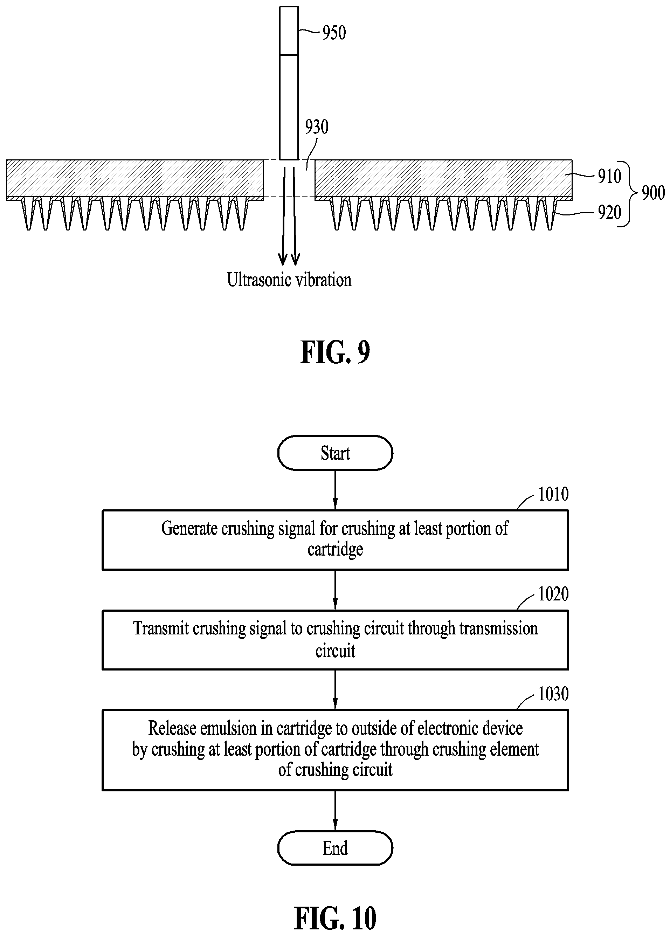

FIG. 9 illustrates a cross-sectional view of an electronic device capable of transmitting external ultrasound in the direction of the skin of a user, according to an example.

According to an embodiment, an electronic device 900 (e.g., the electronic device 100 of FIG. 1 or the electronic device 200 of FIG. 2) may include a body portion 910 and a microneedle portion 920. For example, the body portion 910 may include the user interface 210, the controller 220, the crushing circuit 230, and the cartridge accommodation portion 240.

According to an embodiment, the electronic device 900 may include an ultrasound transmission element 930 through which ultrasound or ultrasonic vibrations may pass from one side (e.g., the upper surface) to the other side (e.g., the bottom surface) of the electronic device 900. For example, the shape of the ultrasound transmission element 930 may be a hole that penetrates the body portion 910 and the microneedle portion 920. For example, the ultrasound transmission element 930 may be an element made of a transparent material formed in the body portion 910 and the microneedle portion 920.

According to an embodiment, a user may irradiate ultrasound emitted by an ultrasound emission device 950 onto one side of the electronic device 900. For example, the ultrasound emission device 950 may be an electronic cigarette or an inhaler. Ultrasound emitted by the ultrasound emission device 950 may reach the other side of the electronic device 900 through the ultrasound transmission element 930 of the electronic device 900. In a state in which the electronic device 900 is attached to the skin of a user, when the user irradiates ultrasound onto the upper surface of the electronic device 900 using the ultrasound emission device 950, the emitted ultrasound may reach the bottom surface of the electronic device 900 and thus reach the skin of the user.

FIG. 10 is a flowchart of a method (not claimed) of releasing emulsion, according to an embodiment.

Operations 1010 to 1030 may be performed by an electronic device (e.g., the electronic device 110 of FIG. 1 or the electronic device 200 of FIG. 2).

In operation 1010, the electronic device may generate a crushing signal for crushing at least a portion (e.g., the first block 371 of FIG. 3A) of a cartridge accommodated in the electronic device.

According to an embodiment, the electronic device may receive a user input from a user through a button (e.g., the button 212 of FIG. 2) of the electronic device and generate the crushing signal based on the user input. For example, the user input may include information (e.g., immediate or a timer) about the time at which the crushing signal is generated and information about the amount of emulsion to be released. For example, a single press of the button by a user may indicate a first emulsion amount, and two presses of the button by the user may indicate a second emulsion amount (e.g., twice the first emulsion amount).

For example, button A may be a button for the user to turn on the electronic device. For example, button B may be a button for the user to input the amount of emulsion (e.g., 0.1 milligrams (mg) when the button is pressed once, 0.3 mg when the button is pressed twice, and 0.5 mg when the button is pressed three times). For example, button C may be a button for the user to input an emulsion release command. According to an example, one button may perform one or more functions. The user may input different commands by pressing a button in different patterns.

According to an embodiment, information input by the user may be visually indicated through an indicator (e.g., the indicator 214 of FIG. 2). For example, the indicator may include a plurality of LEDs, and each LED may indicate a current state (e.g., a power state or emulsion release progress state) of the electronic device or a set amount of emulsion.

According to an embodiment, the electronic device may receive the user input from an external device connected to the electronic device through wireless communication. For example, the external device may include the user terminal 120 (e.g., a smartphone) or the smokeless inhalation device 130 (e.g., an electronic cigarette or an inhaler) of FIG. 1. A method of receiving the user input from the external device is described in detail below with reference to FIG. 11.

According to an embodiment, the electronic device may monitor a state of the user through a sensor of the electronic device disposed on the skin of the user and generate a crushing signal based on a monitoring result. For example, when the sensor of the electronic device is a blood glucose sensor, the electronic device may monitor blood glucose of the user using the blood glucose sensor. When the level of blood glucose of the user is greater than a preset value, the crushing signal of the cartridge may be generated such that emulsion containing insulin is supplied to the user.

According to an embodiment, the crushing signal may be generated such that a preset amount of emulsion is released at a preset time. For example, the time at which the crushing signal is generated by the user may be set in advance. For example, the time may be set in advance such that 0.2 mg of emulsion is released at 8 o'clock, 12 o'clock, and 7 o'clock. For example, when 0.1 mg of emulsion is accommodated in one block of the cartridge, the crushing signal may be generated such that two blocks are crushed at each target time.

According to an embodiment, the generation of the crushing signal may involve supplying power to a closed circuit (e.g., the first closed circuit 391, the second closed circuit 393, and/or the third closed circuit 395 of FIG. 3A) formed by a transmission circuit (e.g., the transmission circuit 225 of FIG. 2) and a crushing circuit (e.g., the crushing circuit 230 of FIG. 2), thereby allowing a current to flow through the closed circuit.

In operation 1020, the electronic device may transmit the generated crushing signal to the crushing circuit through the transmission circuit. For example, the crushing signal may be transmitted to the crushing circuit only through the first closed circuit 391 of a plurality of closed circuits. In this case, power may be supplied only to the first crushing element of the crushing circuit. When power is supplied to the first crushing element, a first bump (e.g., the first bump 361 of FIG. 3A) of the first crushing element may generate heat.

In operation 1030, the electronic device may release the emulsion in the cartridge to the outside by crushing at least one portion (e.g., the first block 371 of FIG. 3B) of the cartridge through a crushing element of the crushing circuit. For example, the emulsion in a block of the crushed cartridge may be released to the outside of the electronic device through a microneedle (e.g., the first microneedle 381 of FIG. 3B) disposed to correspond to the block. When the electronic device is attached to the skin of the user, the emulsion released to the outside of the electronic device may be absorbed into the capillaries of the user.

FIG. 11 is a flowchart of a method (not claimed) of generating a crushing signal based on a user input, according to an example.

According to an embodiment, operation 1110 may be performed before operation 1010 described above with reference to FIG. 10 is performed.

In operation 1110, an electronic device (e.g., the electronic device 110 of FIG. 1 or the electronic device 200 of FIG. 2) may receive a user input.

According to an embodiment, the electronic device may receive the user input through a user interface (e.g., the user interface 210 of FIG. 2) of the electronic device.

According to an embodiment, the electronic device may receive the user input through an external device (e.g., the user terminal 120 (e.g., a smartphone) or the smokeless inhalation device 130 (e.g., an electronic cigarette or an inhaler) of FIG. 1) through a communication unit (e.g., the communication unit 222 of FIG. 2). For example, the communication unit may establish a wireless communication channel with the external device and receive the user input from the external device through the wireless communication channel.

According to an embodiment, a user of an inhaler may set the amount of emulsion to be released through an input device such as a touch/button of the inhaler, and the inhaler may transmit the user input to the electronic device through the wireless communication channel. For example, when inhalation or a puff by the user is detected through the inhaler, the inhaler may transmit a preset user input to the electronic device through the wireless communication channel.

According to an embodiment, a user of a smartphone may input a user input to the electronic device through an application for controlling the electronic device installed on the smartphone. For example, the user may set a time at which emulsion is released, the amount of emulsion, and the like through the application of the smartphone. For example, a doctor associated with the user may transmit the user input to the electronic device through the user terminal such that the emulsion for disease treatment is supplied to the user at the right time and in the appropriate amount.

According to an embodiment, operation 1010 described above with reference to FIG. 10 may include operation 1120 below.

In operation 1120, the electronic device may generate a crushing signal based on a user input. For example, when the user input includes conditions for generating the crushing signal and the conditions are satisfied, the electronic device may generate the crushing signal.

FIG. 12 is a flowchart of a method (not claimed) of controlling an IR lamp, according to an example.

According to an embodiment, operations 1210 and 1220 may be further performed after operation 1030 described above with reference to FIG. 10 is performed. An electronic device (e.g., the electronic device 110 of FIG. 1 or the electronic device 200 of FIG. 2) may perform operations 1210 and 1220.

In operation 1210, the electronic device may generate a first lamp signal that controls a first IR lamp (e.g., the IR lamp 623 of FIG. 6) corresponding to a first block of a cartridge among one or more IR lamps. For example, the first block may be a block of a plurality of blocks of the cartridge that is crushed. When the electronic device includes a plurality of IR lamps, the first lamp signal that controls at least some of the IR lamps corresponding to the first block may be generated. When the electronic device includes one IR lamp, a lamp signal that controls an IR lamp may always be generated regardless of which block emulsion is being released from.

In operation 1220, the electronic device may control the first IR lamp based on the first lamp signal. For example, the first lamp signal may be a signal (e.g., power) supplied to a closed circuit formed through the wires 313 and 314, the third transmission element 323, the fourth transmission element 324, the first electrode 621, the second electrode 622, and the IR lamp 623 described above with reference to FIG. 6. The first IR lamp that receives the signal may emit IR light. For example, IR light may be emitted onto the skin of a user.

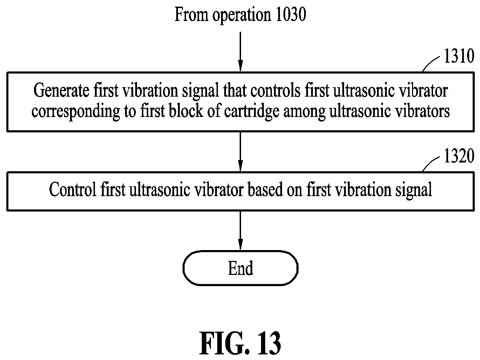

FIG. 13 is a flowchart of a method (not claimed) of controlling an ultrasonic vibrator, according to an example.

According to an embodiment, operations 1310 and 1320 may be further performed after operation 1030 described above with reference to FIG. 10 is performed. An electronic device (e.g., the electronic device 110 of FIG. 1 or the electronic device 200 of FIG. 2) may perform operations 1310 and 1320.

In operation 1310, the electronic device may generate a first vibration signal that controls a first ultrasonic vibrator (e.g., the ultrasonic vibrator 823 of FIG. 8) corresponding to a first block of a cartridge among one or more ultrasonic vibrators. For example, the first block may be a block of a plurality of blocks of the cartridge that is crushed. When the electronic device includes a plurality of ultrasonic vibrators, the first vibration signal that controls at least some of the ultrasonic vibrators corresponding to the first block may be generated. When the electronic device includes one ultrasonic vibrator, a vibration signal that controls the ultrasonic vibrator may be always generated regardless of which block emulsion is being released from.

In operation 1220, the electronic device may control the first ultrasonic vibrator based on the first vibration signal. For example, the first vibration signal may be a signal (e.g., power) supplied to a closed circuit formed through the wires 313 and 314, the third transmission element 323, the fourth transmission element 324, the first electrode 821, the second electrode 822, and the ultrasonic vibrator 823 described above with reference to FIG. 8. The first ultrasonic vibrator that receives the signal may release ultrasound or ultrasonic vibrations. For example, ultrasound or ultrasonic vibrations may be transmitted onto the skin of a user.

The methods according to the embodiments may be recorded in non-transitory computer-readable media including program instructions to implement various operations of the embodiments. The media may also include, alone or in combination with the program instructions, data files, data structures, and the like. The program instructions recorded on the media may be those specially designed and constructed for the purposes of embodiments, or they may be of the kind well-known and available to one of ordinary skill in the computer software arts. Examples of non-transitory computer-readable media include magnetic media such as hard disks, floppy disks, and magnetic tape; optical media such as CD-ROM discs or DVDs; magneto-optical media such as floptical disks; and hardware devices that are specially configured to store and perform program instructions, such as read-only memory (ROM), RAM, flash memory, and the like. Examples of program instructions include both machine code, such as produced by a compiler, and files containing higher-level code that may be executed by the computer using an interpreter. The above-described hardware devices may be configured to act as one or more software modules in order to perform the operations of the embodiments, or vice versa.

The software may include a computer program, a piece of code, an instruction, or one or more combinations thereof, to independently or collectively instruct or configure the processing device to operate as desired. Software and/or data may be embodied permanently or temporarily in any type of machine, component, physical or virtual equipment, computer storage medium or device, or in a propagated signal wave capable of providing instructions or data to or being interpreted by the processing device. The software may also be distributed over network-coupled computer systems so that the software may be stored and executed in a distributed fashion. The software and data may be stored by one or more non-transitory computer-readable recording mediums.

## Claims

1. An electronic device (110) for releasing emulsion, the electronic device (110) comprising:
a cartridge accommodation portion (240) configured to accommodate a cartridge containing emulsion (372) therein;
a crushing circuit (230) comprising a crushing element configured to crush at least a portion of the cartridge disposed in the cartridge accommodation portion (240);
a processor (223) configured to generate a crushing signal; and
a transmission circuit (225) configured to transmit the crushing signal to the crushing circuit (230),
wherein the emulsion (372) is released to an outside of the electronic device (110) by a microneedle (381, 383, 385),
wherein the emulsion (372) is exposed as at least the portion of the cartridge is crushed;
wherein the crushing element is formed in a substrate (350) of the crushing circuit (230) to penetrate the substrate (350), and the crushing element is configured to form a bump (361) on a second surface of the substrate (350) while forming a closed circuit with a first transmission element (321) and a second transmission element (322) of the transmission circuit (225) on a first surface of the substrate (350).

2. The electronic device of claim 1, wherein
a material of the crushing element comprises a material that generates heat by the crushing signal, and
at least the portion of the cartridge is crushed by heat generated by the crushing element.

3. The electronic device (310) of claim 2, wherein the material of the crushing element is a metal having a specific resistance.

4. The electronic device (310) of claim 2, wherein at least the portion of the cartridge crushed by the heat is a thin-film cell.

5. The electronic device (310) of claim 1, wherein the crushing circuit (230) further comprises an elastic element (410) disposed between one side of a substrate (350) of the crushing circuit and the cartridge.

6. The electronic device (310) of claim 5, wherein
the crushing circuit (230) further comprises an emulsion release element (420) attached to the elastic element (410), and
the elastic element (410) and the emulsion release element (420) are configured to move the emulsion (372) in a direction of the microneedle (381, 383, 385) when at least the portion of the cartridge is crushed by the crushing element.

7. The electronic device (310) of claim 1, further comprising an indicator (214) indicating an amount of emulsion (372) to be released.

8. The electronic device (310) of claim 1, further comprising a button (212) configured to receive, from a user (101), an amount of emulsion (372) to be released.

9. The electronic device (310) of claim 1, wherein the processor (223) is configured to, when the cartridge comprises a plurality of blocks (371), each containing emulsion (372), generate the crushing signal to crush one or more blocks (371) of the plurality of blocks (371).

10. The electronic device (310) of claim 9, wherein
the crushing circuit (230) comprises a plurality of crushing elements, and
the plurality of crushing elements respectively corresponds to the plurality of blocks (371).

## Patentansprüche

1. Elektronische Vorrichtung (110) zum Freisetzen einer Emulsion, wobei die elektronische Vorrichtung (110) Folgendes umfasst:
einen Kartuschenaufnahmeabschnitt (240), der konfiguriert ist, eine Kartusche aufzunehmen, in der eine Emulsion (372) enthalten ist;
eine Brechschaltung (230), die ein Brechelement umfasst, das konfiguriert ist, zumindest einen Abschnitt der Kartusche, die im Kartuschenaufnahmeabschnitt (240) angeordnet ist, zu brechen;
einen Prozessor (223), der konfiguriert ist, ein Brechsignal zu erzeugen; und
eine Sendeschaltung (225), die konfiguriert ist, das Brechsignal an die Brechschaltung (230) zu senden,
wobei die Emulsion (372) durch eine Mikronadel (381, 383, 385) in die äußere Umgebung der elektronischen Vorrichtung (110) freigesetzt wird,
wobei die Emulsion (372) freigesetzt wird, wenn zumindest der Abschnitt der Kartusche gebrochen wird;
wobei das Brechelement in einem Substrat (350) der Brechschaltung (230) gebildet ist, derart, dass es das Substrat (350) durchdringt, und das Brechelement konfiguriert ist, einen Höcker (361) auf einer zweiten Fläche des Substrats (350) zu bilden, während es auf einer ersten Fläche des Substrats (350) eine geschlossene Schaltung mit einem ersten Sendeelement (321) und einem zweiten Sendeelement (322) der Sendeschaltung (225) bildet.

2. Elektronische Vorrichtung nach Anspruch 1, wobei
ein Material des Brechelements ein Material umfasst, das durch das Brechsignal Wärme erzeugt, und
zumindest der Abschnitt der Kartusche durch Wärme, die durch das Brechelement erzeugt wird, gebrochen wird.

3. Elektronische Vorrichtung (310) nach Anspruch 2, wobei das Material des Brechelements ein Metall ist, das einen bestimmten Widerstand aufweist.

4. Elektronische Vorrichtung (310) nach Anspruch 2, wobei zumindest der Abschnitt der Kartusche, der durch die Wärme gebrochen wird, eine Dünnschichtzelle ist.

5. Elektronische Vorrichtung (310) nach Anspruch 1, wobei die Brechschaltung (230) ferner ein elastisches Element (410) umfasst, das zwischen einer Seite eines Substrats (350) der Brechschaltung und der Kartusche angeordnet ist.

6. Elektronische Vorrichtung (310) nach Anspruch 5, wobei
die Brechschaltung (230) ferner ein Emulsionsfreisetzungselement (420) umfasst, das am elastischen Element (410) befestigt ist, und
das elastische Element (410) und das Emulsionsfreisetzungselement (420) konfiguriert sind, die Emulsion (372) in einer Richtung der Mikronadel (381, 383, 385) zu bewegen, wenn zumindest der Abschnitt der Kartusche durch das Brechelement gebrochen wird.

7. Elektronische Vorrichtung (310) nach Anspruch 1, die ferner eine Anzeige (214) umfasst, die eine freizusetzende Menge der Emulsion (372) anzeigt.

8. Elektronische Vorrichtung (310) nach Anspruch 1, die ferner ein Bedienelement (212) umfasst, das konfiguriert ist, von einem Benutzer (101) eine Menge der freizusetzenden Emulsion (372) zu empfangen.

9. Elektronische Vorrichtung (310) nach Anspruch 1, wobei der Prozessor (223) konfiguriert ist, dann, wenn die Kartusche mehrere Blöcke (371) umfasst, die jeweils Emulsion (372) enthalten, das Brechsignal zu erzeugen, um einen oder mehrere Blöcke (371) der mehreren Blöcke (371) zu brechen.

10. Elektronische Vorrichtung (310) nach Anspruch 9, wobei
die Brechschaltung (230) mehrere Brechelemente umfasst und
die mehreren Brechelemente jeweils den mehreren Blöcken (371) entsprechen.

## Revendications

1. Dispositif électronique (110) destiné à libérer une émulsion, le dispositif électronique (110) comportant :
une partie de réception de cartouche (240) configurée pour recevoir une cartouche contenant une émulsion (372) dans celle-ci ;
un circuit d'écrasement (230) comportant un élément d'écrasement configuré pour écraser au moins une partie de la cartouche disposée dans la partie de réception de cartouche (240) ;
un processeur (223) configuré pour générer un signal d'écrasement ; et
un circuit de transmission (225) configuré pour transmettre le signal d'écrasement au circuit d'écrasement (230),
dans lequel l'émulsion (372) est libérée vers un extérieur du dispositif électronique (110) par une micro-aiguille (381, 383, 385),
dans lequel l'émulsion (372) est exposée lorsqu'au moins la partie de la cartouche est écrasée ;
dans lequel l'élément d'écrasement est formé dans un substrat (350) du circuit d'écrasement (230) pour pénétrer dans le substrat (350), et l'élément d'écrasement est configuré pour former une bosse (361) sur une seconde surface du substrat (350) tout en formant un circuit fermé avec un premier élément de transmission (321) et un second élément de transmission (322) du circuit de transmission (225) sur une première surface du substrat (350).

2. Dispositif électronique selon la revendication 1, dans lequel
un matériau de l'élément d'écrasement comporte un matériau qui génère de la chaleur par le signal d'écrasement, et
au moins la partie de la cartouche est écrasée par la chaleur générée par l'élément d'écrasement.

3. Dispositif électronique (310) selon la revendication 2, dans lequel le matériau de l'élément d'écrasement est un métal ayant une résistance spécifique.

4. Dispositif électronique (310) selon la revendication 2, dans lequel au moins la partie de la cartouche écrasée par la chaleur est une cellule à couches minces.

5. Dispositif électronique (310) selon la revendication 1, dans lequel le circuit d'écrasement (230) comporte en outre un élément élastique (410) disposé entre un côté d'un substrat (350) du circuit d'écrasement et la cartouche.

6. Dispositif électronique (310) selon la revendication 5, dans lequel
le circuit d'écrasement (230) comporte en outre un élément de libération d'émulsion (420) fixé à l'élément élastique (410), et
l'élément élastique (410) et l'élément de libération d'émulsion (420) sont configurés pour déplacer l'émulsion (372) dans une direction de la micro-aiguille (381, 383, 385) lorsqu'au moins la partie de la cartouche est écrasée par l'élément d'écrasement.

7. Dispositif électronique (310) selon la revendication 1, comportant en outre un indicateur (214) indiquant une quantité d'émulsion (372) à libérer.

8. Dispositif électronique (310) selon la revendication 1, comportant en outre un bouton (212) configuré pour recevoir, d'un utilisateur (101), une quantité d'émulsion (372) à libérer.

9. Dispositif électronique (310) selon la revendication 1, dans lequel le processeur (223) est configuré pour, lorsque la cartouche comporte une pluralité de blocs (371), contenant chacun une émulsion (372), générer le signal d'écrasement afin d'écraser un ou plusieurs blocs (371) de la pluralité de blocs (371).

10. Dispositif électronique (310) selon la revendication 9, dans lequel
le circuit d'écrasement (230) comporte une pluralité d'éléments d'écrasement, et la pluralité d'éléments d'écrasement correspond respectivement à la pluralité de blocs (371).
